Europäisches Patentamt

European Patent Office

Office européen des brevets

(11) Publication number: 0 209 036
A1

## EUROPEAN PATENT APPLICATION

(21) Application number: 86109252.6

(51) Int. Cl.⁴: **A61K 6/00**

(22) Date of filing: 24.03.81

(30) Priority: 07.04.80 US 137631
25.07.80 US 172218

(43) Date of publication of application:
21.01.87 Bulletin 87/04

(60) Publication number of the original application in
accordance with Art.76 EPC: 0 037 677

(84) Designated Contracting States:
CH DE FR GB IT LI

(71) Applicant: MINNESOTA MINING AND
MANUFACTURING COMPANY
3M Center, P.O. Box 33427
St. Paul, Minnesota 55133-3427(US)

(72) Inventor: Randklev, Ronald M.
2501, Hudson Road P.O. Box 33427
Saint Paul Minnesota 55133(US)

(74) Representative: Baillie, Iain Cameron et al
c/o Ladas & Parry Isartorplatz 5
D-8000 München 2(DE)

(54) A dental abrasive composition.

(57) A dental abrasive composition comprising a fine-
ly divided abrasive filler admixed with a solid binder,
wherein the abrasive filler is a non-toxic inorganic
material having a Mohs hardness less than 5, and
being at least 30% of the total weight of the abrasive
composition. The dental abrasive composition of this
invention can be used in a method and/or a kit for
applying to and removing from a tooth surface an
orthodontic bracket as described in co-pending European Patent Application No. 81301258.6

EP 0 209 036 A1

## A DENTAL ABRASIVE COMPOSITION

Technical Field

This invention relates to a dental abrasive composition. The abrasive composition of this invention can be used in a method and/or a kit for applying to and removing from a tooth surface an orthodontic bracket as described in co-pending European Patent Application No. 81301258.6.

Background Art

As described in co-pending European Patent Application No. 81301258.6 the fillers used in orthodontic bracket adhesives impart a sufficient viscosity to the adhesive to make it readily workable in the mouth. Without such a filler, the adhesive or the bracket is prone to slippage prior to cure. The fillers used in orthodontic bracket adhesives are generally also used in some dental restorative resins and include quartz, silica gel, aluminum silicate, silica, glass beads, aluminum oxide, titanium dioxide, zirconia, spodumene, lithium aluminum silicate, barium aluminum silicate, and silicate or phosphate glasses, see U.S. Patent Nos. 3,625,916, 3,629,187, 3,709,866, 3,895,445, 4,010,545, and 4,063,360, and U.K. Published Patent Application 2,006,792 A. Some references have suggested using lower amounts of filler in an orthodontic bracket adhesive than the amounts typically used in those restorative resins containing such fillers in order to ease removal of excess adhesive, see Gwinnett and Gorelick, id, Retief and Denyls id, and U.S. Patent No. 3,629,187. However, removal of residual adhesive containing these fillers still can lead to damage to tooth enamel, because hardened tools must be used to grind away the excess adhesive.

The hardness of tooth enamel, measured upon the Mohs scale of hardness, is approximately 4.5 to 5, depending upon the age and location of the tooth. The fillers used in currently marketed orthodontic bracket adhesives have a Mohs hardness well in excess of this value. Consequently, yet harder abrasives must be used to remove such adhesives. Currently used abrasives include silicon carbide, diamond, stainless steel, aluminum oxide, and pumice. Such abrasives are described, for example, in Greener, Harcourt and Lautenschlager, "Materials Science in Dentistry", Williams & Wilkins, Baltimore (1972) at, e.g. pp. 379-383, Craig, O'Brien and Powers, "Dental Materials, Properties and Manipulation" Mosby, St. Louis (1979) at e.g. Chapter 6, and Anderson, "Applied Dental Materials", Blackwell, Oxford (1972) at, e.g. Chapter 29. All of these abrasives readily abrade tooth enamel.

In U.S. Patent No. 4,141,144 (Lustgarten) there are described compositions for use as "paint-on" tooth veneers and direct filling materials, said to be useful for matching the hue and lustre of treated teeth with the hue and lustre of untreated teeth. Such compositions contain about 1 to 85 percent by weight muscovite mica flakes having an average size of less than about 50 micrometers. In such compositions some or all of the muscovite mica flakes are preferably coated with a continuous layer of $TiO_2$ or $ZrO_2$ (which coating materials have Mohs hardnesses of 5-½ to 6-½ and 7 to 7-½ respectively), or BiOCl (which coating material has an unreported Mohs hardness, due perhaps to the commercial unavailability of crystalline BiOCl in a crystal size sufficient to allow Mohs hardness testing to be carried out). Also, such compositions preferably contain inorganic filler such as silica, glass beads, aluminum oxide, fused silica, fused or crystalline quartz, or the like (which inorganic fillers have generally high Mohs hardnesses, e.g., 7 or more). Such compositions therefore preferably contain inorganic materials whose surfaces are harder than tooth enamel. In the only example in Lustgarten in which uncoated mica is used and coated mica and inorganic filler are excluded (viz., Example 14) about 22 percent by volume (about 39 to 41 percent by weight) uncoated muscovite mica was combined with methyl methacrylate to form a thin, yellowish 1.5 mm thick cured slab which was used in various optical comparison tests.

Dental restorative resins containing a filler having a Mohs hardness of 3 to 5 have been described as offering good wear resistance and polishability in U.S. Patent No. 4,020,557, and dental restorative resins containing a rod-like filler having a Mohs hardness of 3.5 to 6 (such as calcium silicate) have been described as offering a resin with good strength and workability in Australian Patent Specification No. 50674/73. These references do not suggest the use of such fillers in an orthodontic bracket adhesive and abrasive system.

The above cited U.K. Published Patent Application No. 2,006,792 A states that an organic filler such as polymethyl methacrylate can be used in an orthodontic bracket adhesive. Such an organically filled adhesive has low hardness but insufficient tensile strength.

Disclosure of Invention

The present invention provides a dental abrasive composition which can be used in a method and/or a kit for applying to and removing from a tooth surface an orthodontic bracket.

The dental abrasive composition, characterised in that said abrasive composition comprises a finely divided abrasive filler admixed with a solid binder, said abrasive filler being non-toxic and having a Mohs hardness less than 5, and with said abrasive filler being at least 30% of the total weight of said abrasive composition.

Detailed Description

The present invention provides an abrasive which removes the adhesive as described in co-pending European Patent Application No. 81301258.6 but does not damage tooth enamel. The abrasive has a Mohs hardness greater than the Mohs hardness of the filler used in the bracket adhesive but less than about 5. Preferably the abrasive has a Mohs hardness less than about 4.5, in order to avoid damaging the enamel of even the softest teeth. Most preferably, the abrasive has a Mohs hardness well below that of enamel but more than about 1 Mohs hardness unit greater than the Mohs hardness of the adhesive filler. For example, if an adhesive filler with a Mohs hardness less than about 2 is used, then the abrasive used should have a Mohs hardness of about 3.

Also, the abrasive is non-toxic, but this requirement is less stringent than the toxicity requirement for the adhesive filler due to the brief patient exposure involved when such abrasives are used. In addition, it is desirable that the abrasive be essentially insoluble in mouth fluids, but this is not essential in the practice of this invention since the abrasive filler may be worn away in use and rinsed out of the mouth before it extensively dissolves.

The abrasive can be used in solid form as a scraping or cutting tool (such as a file, rasp, or knife) or the abrasive can be finely divided, mixed with a suitable solid binder, and formed into solid, shaped, composite tools such as files, rasps, or grinding attachments for standard powered dental tools. Such grinding attachments come in a variety of shapes but all have a central axis of symmetry and attaching means (such as a mandrel or an adhesive backing) for chucking them into a powered dental tool. Preferably the abrasives of this invention are used in the form of composite grinding attachments for a dental tool, because such attachments can be operated very rapidly, maximizing patient comfort and economizing on use of the orthodontist's time.

When the abrasive is in the form of a composite tool containing finely divided abrasive particles mixed with a binder, the binder can be any of the materials commonly used for dental abrasive tools of the prior art. These binders are well known to those skilled in the art and include materials such as phenolic resins and epoxies. Preferred binders for use in the present invention include cross-linked, rigid polymeric materials such as cured phenolic, epoxy, polyester, and polyurethane resins and soft materials such as silicone and neoprene rubbers.

Also, when the abrasive is in the form of a composite tool containing finely divided abrasive particles mixed with a binder, the abrasive should represent greater than about 30% by weight of the total weight of abrasive and binder, preferably greater than about 50% by weight, and most preferably about 60 to about 70% by weight. The abrasive filler should be pulverized or comminuted in order to facilitate thorough mixing of the abrasive into the binder. Relatively large abrasive filler particles (e.g. 190 micrometers or greater mean diameter) are preferred, in order to yield a tool having a high cutting rate. It should be noted that this differs from current dental abrasive practice, where small diameter filler particles have been recommended in order to avoid causing deep scratches in tooth enamel.

In addition, where composite grinding attachments are prepared by finely dividing an abrasive, mixing it with a binder, and coating the mixture onto a flexible backing such as a polymer web (e.g. in the production of small abrasive disks which will be coated with an adhesive backing and applied to a suitable mandrel), then the shape of the abrasive particles is important. It is preferred that such abrasive particles have shapes which are generally equiaxed, rather than acicular, fibrous, or otherwise excessively elongated. Using ordinary coating methods, elongated particles tend to align themselves with the plane represented by the web and become overcoated with binder resin. Abrasive disks made from such particles have poor abrasive ability. On the other hand, equiaxed particles tend to randomly orient themselves at the surface of the tool, and protrude through the binder surface, exposing optimum cutting edges. Abrasive disks made from such equiaxed particles have good abrasive ability, and can be more effective grinding attachments than attachments made from abrasive particles which are harder but elongated in shape.

Several suitable abrasives are set out below in Table I. Data regarding chemical composition and hardness are from Hurlbut, Jr. and Klein, Manual of Mineralogy, id , except where otherwise indicated.

<center>TABLE I</center>

| Abrasive filler | Composition | Mohs hardness |
|---|---|---|
| Aragonite | $CaCo_3$ | 3 1/2 - 4 |
| Apatite | $Ca_5(PO_4)_3(F, Cl, OH)$ | 5 |
| Apophyllite | $KCa_4(Si_4O_{10})_2F \cdot 8H_2O$ | 4 1/2 - 5 |
| Barite | $BaSO_4$ | 3 - 3 1/2 |
| Boehmite | $AlO(OH)$ | 3 1/2 - 4 |
| Calcite | $CaCO_3$ | 3 |
| Colemanite | $CaB_3O_4(OH)_3 \cdot H_2O$ | 4 - 4 1/2 |
| Dolomite | $CaMg(CO_3)_2$ | 3 1/2 - 4 |
| Fluorite | $CaF_2$ | 4 |
| Gibbsite | $Al(OH)_3$ | 2 1/2 - 3 1/2 |
| Hectorite | $(Mg, Li)_3Si_4O_{10} - (OH)_2Na_{0.3}(H_2O)_4$ | 1 - 1 1/2 |
| Hemimorphite | $Zn_4(Si_2O_7)(OH)_2 \cdot H_2O$ | 4 1/2 - 5 |
| Hopeite* | $Zn_3(PO_4)_2 \cdot 4H_2O$ | 3 1/4 |
| Kaolinite | $Al_2Si_2O_5(OH)_4$ | 2 |
| Langbeinite | $K_2Mg_2(SO_4)_3$ | 3 1/2 - 4 |
| Marble | Predominately $CaCO_3$ | 3 - 4 |
| Muscovite | $KAl_2(AlSi_3O_{10})(OH)_2$ | 2 - 2 1/2 |
| Parisite* | $(Ce, La, Na,)FCO_3 \cdot CaCO_3$ $FCO_3 \cdot CaCo_3$ | 4 1/2 |
| Phlogopite | $KMg_3(AlSi_3O_{10})(OH)_2$ | 2 1/2 - 3 |
| Scheelite | $CaWO_4$ | 4 1/2 - 5 |
| Smithsonite | $ZnCO_3$ | 4 - 4 1/2 |
| Sphalerite | $ZnS$ | 3 1/2 - 4 |
| Wollastonite** | $CaSiO_3$ | 4 - 5 1/2 |
| Zincite | $ZnO$ | 4 |

*Handbook of Chemistry and Physics, 50th Ed., Chemical Rubber Co., Cleveland (1969).

** Hurlbut and Klein list the hardness of Wollastonite as 5-5 1/2. Values as low as 4 have been reported - see, e g. Kraus, Hunt, and Ramsdell, Mineralogy Manual, McGraw Hill (1936), and 4.5 - see, e.g., U.S. Patent No. 4,020,557.

Mixtures of more than one abrasive can be used in this invention. Calcite, dolomite, and marble are preferred abrasives, with marble being most preferred. A light-colored marble known as "white Georgia marble , a material having a Mohs hardness of about 3 and a composition which is approximately 97% $CaCO_3$/ 3% $MgCO_3$, has been found to give very good results in this invention. White Georgia marble is similar in chemical compositions to calcite (pure crystalline $CaCO_3$) and dolomite (variable $CaCO_3/MgCO_3$ composition). White Georgia marble fractures with rhombohedral cleavage, and is especially effective when finely divided and mixed with a binder. The effect of abrasive particle shape may be further understood by noting that composite grinding attachments made by coating a mixture of finely divided white Georgia marble and a binder onto a web have a faster cutting rate than similar attachments made from harder but more elongated materials such as wollastonite. Wollastonite fractures with cleavage ranging from an acicular to a fibrous shape. As described above, such elongated particles tend to lie down on the web rather than protrude, and therefore are less effective as an abrasive than rhombohedral abrasive particles of white Georgia marble, even though wolastonite has a Mohs hardness of 5 to 5.5 while white Georgia marble has a Mohs hardness of 3. Other adjuvants (e.g., pigments) can also be used in the abrasives of this invention. Such adjuvants, if solid, preferably are no harder than tooth enamel. The use of adjuvants is not required in the abrasives of this invention, as useful abrasives can be prepared which consist of or consist essentially of non-toxic inorganic material having a Mohs hardness less than about 5 and solid binder.

The abrasives of this invention are typically used at the completion of orthodontic treatment of a tooth. An orthodontic bracket which has been adhered to the tooth by means of the adhesives described above is first removed from the tooth surface using orthodontic pliers or ligature cutters. Next, the residual adhesive remaining on the tooth surface is removed by grinding or scraping. Preferably residual adhesive is removed within as short a time as possible without excessive damage to the enamel. Complete removal within about 15 seconds can be regarded as a very satisfactory removal time.

It has been found that the abrasives of this invention are also useful in general dental work. These abrasives are surprisingly effective at rapidly cutting, polishing, and otherwise working dental composite materials filled with very hard fillers such as quartz (which has a Mohs hardness of about 7). Ordinarily, this would not be expected due to the low hardness of the abrasives in this invention and the high hardness of the fillers typically used in such dental composite materials. It is theorized that the abrasives of this invention cut into the polymerizable resins used in such dental composite materials, and the hard filler granules in those materials then fall or pop out. Accordingly, the abrasives of this invention used alone as a general tool for working composite dental materials such as dental restorative resin, acrylic teeth, and denture bases, constitute an additional aspect of this invention.

The present invention therefore provides an abrasive useful for removing orthodontic brackets.

The following examples are offered to aid understanding of the present invention and are not to be construed as limiting the scope thereof.

Example 1

Marble Abrasive Disks

Five 50 gm portions of white Georgia marble chips (commercially available from Georgia Marble Company) were crushed and screened to the following size fractions in separate samples:

600 to 425 micrometers

425 to 250 micrometers

250 to 150 micrometers

150 to 106 micrometers

106 to 74 micrometers.

Each sample was then mixed with a solution of 49 gms of "Bakelite PKHH" phenoxy resin - (commercially available from Union Carbide Corp.) in 114 gms of methylethylketone, together with 1 gm of "PAPI 100" polymethylene polyphenylisocyanate (commercially available from Upjohn Co.). These ingredients were mixed by mechanical mixing and then knife coated (using a 171 micrometer gap) onto a silicon dioxide coated 190 micrometer thick polyester film (commercially available from Teledyne Post Inc.). The coated film was then oven cured for 2 hours at 79°C. 15.3 millimeter diameter disks were cut from the cured film, punctured through the center, and then fastened to a brass eyelet by inserting the eyelet through the punctured hole and staking the eyelet to form a square hub. The resulting abrasive disks were fastened to a mandrel which has been mounted in a standard dental hand engine No. 92N, commercially available from Teledyne Emesco Co.). Cured samples 6 mm x 6 mm x 37 mm in size prepared from the adhesives of Examples 1 through 7 were abraded for 15 seconds with the above abrasive disks, using average hand pressure and a rotational speed of 10,000 rpm. The fastest cutting rates were observed with disks made from large marble granules. A disk containing 425 to 600 micrometer abrasive particles removed 49 mg of the adhesive of Example 1. This data demonstrates that the abrasives of this invention rapidly remove the adhesives of this invention.

In a separate test, a 6 mm x 6 mm x 37 mm sample slug was prepared from a conventional quartz filled orthodontic bracket adhesive ("Concise 1960", commercially available from 3M). Under the test conditions described above, 12 mg of adhesive were ground away by a disk containing 425 to 600 micrometer abrasive particles. This illustrated that the abrasives of this invention could be used to cut and polish an orthodontic bracket adhesive filled with a material which is harder than the abrasives of this invention.

In a separate test, a 6 mm x 6 mm x 37 mm sample slug was prepared from a conventional quartz filled dental restorative resin ("Concise 1925", commercially available from 3M). Under the test conditions described above, 10 mg of restorative resin were ground away by a disk containing 425 to 600 micrometer abrasive particles. This illustrated that the abrasive disks of this invention could be used to cut and polish a dental restorative resin filled with a material which is harder than the abrasives of this invention.

Example 2

Wollastonite Abrasive Disks

Using the method of Example 1, finely divided wollastonite ("Nycor 100", commercially available from Interpace Corp.) was combined with phenoxy resin binder and coated onto polyester film. Samples were tested as in Example 8. Again, the largest diameter particles gave highest cutting rates. However, using the same mesh size, wollastonite was roughly half as effective an abrasive as white Georgia marble.

Example 3

Preparation of a Hard Abrasive Wheel

15 gms of white Georgia marble chips, sized on a sieve to 58 to 100 micrometers diameter, were mixed with a solution containing 80 gms of the phenoxy resin of Example 8 and 5 gms of polymethylene polymethylisocyanate. These ingredients were stirred to form a homogeneous mixture and poured into molds to make 13 mm diameter abrasive wheels having a thickness of 1.6 mm. The molded wheels were cured by heating in an oven for 30 minutes at 93°C followed by heating for 14 hours at 60°C. The abrasive wheels were mounted in a powered dental hand tool and tested, using the method of Example 8, by abrading them against a 6 mm X 6 mm x 37 mm sample slug of cured adhesive prepared from the composition of Example 1. 15 second contact using average hand pressure at a rotational speed of 10,000 rpm removed 11.2 mgs of adhesive from the sample slug.

In a separate test, a 6 mm x 6 mm x 37 mm sample slug was prepared from "Concise 1925". Under the test conditions described above, 3.1 mg of restorative resin were ground away by the abrasive wheel. This illustrated that the abrasive wheels of this invention could be used to cut and polish a dental restorative resin filled with a material which is harder than the abrasives of this inventions.

Example 4

Preparation of a Soft Rubber Abrasive Wheel

Fifty gms of white Georgia marble chips sized between 240 and 400 micrometers were combined with 50 gms of neoprene rubber (commercially available from Pawling Rubber Co.) on a 2 roll mill equipped with heat exchange means set at 12-16°C. The milled mixture was formed into a 1.6 mm thick sheet and cured for 8 hours at 150°C. Soft wheels having a diameter of 13 mm were punched from the cured sheet.

The soft wheels were mounted on a mandrel and tested by abrading them against 6 mm x 6 mm x 37 mm sample slugs using the test conditions described in Example 8. The soft wheels removed 24 mg of the adhesive of Example 1, 17 mg of the adhesive of Example 4, and 14 mg of the adhesive of Example 6.

In a separate test, a 6 mm x 6 mm x 37 mm sample slug was prepared from "Concise 1960", quartz filled orthodontic bracket adhesives. Under the test conditions described above, 25 mg of bracket adhesive were ground away by the soft wheel.

Various modifications and alterations of this invention will be apparent to those skilled in the art without departing from the scope and spirit of this invention and the latter should not be restricted to that set forth herein for illustrative purposes.

## Claims

1. A dental abrasive composition, characterised in that said abrasive composition comprises finely divided abrasive filler admixed with a solid binder, said abrasive filler being non-toxic and having a Mohs hardness less than 5, and with said abrasive filler being at least 30% of the total weight of said abrasive composition.

2. A dental abrasive composition according to claim 1, further characterised in that said abrasive filler is in the form of generally equiaxed particles having a mean diameter of at least 190 micrometers, is at least 50% of the total weight of said composition, and is selected from the group consisting of calcite, dolomite and marble.

3. A dental abrasive composition according to either of claims 1 and 2, further characterised in that abrasive filler is formed into a dental tool.

4. A dental abrasive composition according to claims 1 to 3, usable in a method for applying to and removing from a tooth surface an orthodontic bracket in which said abrasive composition is used to remove the bracket.

5. A dental abrasive composition according to claims 1 to 3, usable in a kit for applying to and removing from a tooth surface an orthodontic bracket in which said abrasive composition is used to remove the bracket.

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | GB-A-1 293 727 (INDIANA UNIVERSITY FOUNDATION) * Page 2, line 40 - page 4, line 87; figures * | 1,3-5 | A 61 K 6/00 A 61 C 3/06 |
| Y | | 2,3 | |
| | --- | | |
| Y | DE-A-2 751 135 (FUJI PHOTO FILM CO. LTD.) * Claims 1,5,6,8; page 5, line 14 - page 6, line 1; page 8, line 6 - page 10, line 12 * | 2 | |
| | --- | | |
| X | DE-A-1 912 466 (COMPOLITH ANSTALT) * Page 1, lines 1-19; pages 4-7, example 2; claims 31-34 * | 1,2,4,5 | |
| | | | TECHNICAL FIELDS SEARCHED (Int. Cl.4) |
| Y | | 3 | A 61 K A 61 C C 08 K |
| | --- | | C 08 L |
| X | GB-A- 925 625 (YVES DU TERTRE) * Page 1, lines 1-51; claim 1 * | 1,4,5 | |
| | ----- | | |

The present search report has been drawn up for all claims

| Place of search | Date of completion of the search | Examiner |
|---|---|---|
| THE HAGUE | 31-10-1986 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82